Europäisches Patentamt

European Patent Office

Office européen des brevets

(19)

(11) Publication number: **0 113 373 B1**

(12) **EUROPEAN PATENT SPECIFICATION**
published in accordance with Art.
158(3) EPC

(45) Date of publication of patent specification: **28.08.91**   (51) Int. Cl.⁵: **A61K 9/52, A61K 9/62**

(21) Application number: **83902590.5**

(22) Date of filing: **08.07.83**

(86) International application number:
**PCT/US83/01042**

(87) International publication number:
**WO 84/00295 (02.02.84 84/03)**

The file contains technical information submitted
after the application was filed and not included in
this specification

(54) **SUSTAINED RELEASE QUINIDINE DOSAGE FORM.**

(30) Priority: **09.07.82 US 396669**

(43) Date of publication of application:
**18.07.84 Bulletin 84/29**

(45) Publication of the grant of the patent:
**28.08.91 Bulletin 91/35**

(84) Designated Contracting States:
**AT BE CH DE FR GB LI LU NL SE**

(56) References cited:
**EP-A- 0 063 014       EP-A- 0 067 595
WO-A-83/00284       AU-A- 109 438
US-A- 2 853 420       US-A- 2 887 440
US-A- 2 928 770       US-A- 3 247 066
US-A- 3 344 029       US-A- 3 400 185
US-A- 3 632 739       US-A- 3 773 920
US-A- 3 835 221       US-A- 3 917 813**

(73) Proprietor: **Key Pharmaceuticals, Inc.
2000 Galloping Hills Road
Kenilworth New Jersey 07033(US)**

(72) Inventor: **HSIAO, Charles
4890 104th Avenue
Cooper City, FL 33328(US)**

(74) Representative: **Werner, Hans-Karsten, Dr. et
al
Deichmannhaus am Hauptbahnhof
W-5000 Köln 1(DE)**

US-A- 3 981 984    US-A- 4 016 254
US-A- 4 083 949    US-A- 4 173 626
US-A- 4 321 253    US-A- 4 341 759

CHEMICAL ABSTRACTS, vol. 90, no. 6, February 1979, page 307, column 2, abstract 43776e, Columbus, Ohio, US; P.A. SADO et al.: "Bioavailability of quinidine in prolonged action oral forms", & EXPO.-CONGR. INT. TECHNOL. PHARM. 1ST 1977, 4, 192-203

P.H. LIST et al.: "Hagers Handbuch der pharmazeutischen Praxis", vol. VII, part B, 4th edition, 1977, page 141, Springer Verlag, Berlin, DE;

Chemical Abstracts, Vol. 98, No. 20, issued 16 May 1983 Columbus, Ohio, USA) Hsiao, H.C. (Key Pharmaceuticals, Inc.) PCT Intl. Appl. WO 83,00284, 03 February 1983, Abstract No. 166917

## Description

Summary of the Invention

Quinidine is a well known alkaloid used in the treatment of certain cardiac arrhythmias. It is a general cardiac depressant, which reduces myocardial excitability, automaticity and conductivity.

In accordance with the present invention there is provided a sustained release quinidine dosage form which which comprises a container having therein a plurality of pellets having an average diameter not greater than 1 mm. Each pellet has a coating of quinidine over a nonpareil seed. The quinidine-coated nonpareils are then coated with a mixture of two and one third to nine parts by weight ethylcellulose to one part by weight hydroxypropylcellulose.

The present invention provides a sustained release quinidine dosage form, typically, a capsule containing from 2,000 to 15,000 of the polymer-coated pellets. Each of the pellets is based upon a nonpareil, which is typically a sugar seed having a particle size of 0.7 to 0.85 mm (20 to 25 mesh).

The nonpareils are coated with more than an equal amount by weight of a quinidine compound, which preferably is quinidine bisulfate. In a preferred embodiment, about two parts by weight quinidine bisulfate are coated onto one part by weight of nonpareil seeds. In one embodiment, 500 g nonpareil seeds are placed in a coating pan and wetted with a 30% sucrose solution with the aid of a sprayer. Approximately 30 g of quinidine bisulfate are dusted onto the thus-treated pellets, distributing the material manually as necessary. Wetting and dusting procedures are repeated until a buildup of 1000 g of quinidine bisulfate takes place per 500 g of the nonpareils.

When coated with a greater than equal weight of quinidine, the thus quinidine-coated nonpareil seeds are coated with a sustained release polymeric coating mix, which comprises from 2 1/3 to 9 parts by weight of ethyl cellulose to one part by weight hydroxypropylcellulose.

In one embodiment, 700 g of quinidine bisulfate-nonpareils (2:1 weight ratio), produced above, are charged into an air suspension coating column (Wurster column, 15,24 cm (six inches), manufactured by Glatt Air Techniques) and after commencement of the air suspension of these pellets, there is charged into the air suspension a spray of methanol-chloroform (380 ml methanol; 1400 ml chloroform) which contains 66.8 mg mixture of ethylcellulose (Ethocel® type 10, Dow Chemical Co.) and hydroxypropylcellulose (Klucel® LF, Hercules), this mixture having a weight ratio of 7 parts ethylcellulose to 3 parts hydroxypropylcellulose.

Typical conditions useful for preparing the polymeric coating of the pellets in the above-described equipment include a 40° C inlet temperature, 2 bar spray air pressure and a liquid feed rate of 12 $cm^3$ per minute.

Pellets produced in accordance with the procedures set forth above yield a sustained release over a prolonged period of time. When tested according to U.S.P. XX dissolution procedure (one hour in simulated gastric fluid followed by simulated intestinal fluid) the following release characteristics are noted:

| Time (h) | Percent Release |
|---|---|
| 1 | 6.0 |
| 2 | 20.1 |
| 4 | 43.1 |
| 6 | 64.5 |
| 8 | 84.1 |
| 10 | 96.1 |
| 12 | 99.9 |

The formulation of the present invention is particularly advantageous for pediatric and geriatric patients who may be either unable or unwilling to swallow a larger sustained release tablet. For pediatric administration it is particularly contemplated that the individually coated polymeric pellets are administered in food.

## Claims

3

1. A sustained release quinidine dosage form which comprises a container having therein a plurality of pellets, the average pellet diameter being not greater than 1 mm, each pellet comprising a coating of quinidine over a nonpareil seed, the thus quinidine-coated nonpareils being coated thereon with a mixture of 2 1/3
   to 9 parts by weight ethylcellulose to 1 part by weight hydroxypropylcellulose, the sum of such pellets in a container forming a single dosage unit of quinidine.

2. A sustained release quinidine dosage form of claim 1 wherein said quinidine is in the form of quinidine bisulfate.

3. A sustained release quinidine dosage form of claim 1 wherein the weight of quinidine is approximately twice that of the nonpareil.

4. A sustained release quinidine dosage form of claim 1 wherein said container is a capsule.

**Revendications**

1. Forme de dosage de quinidine à libération entretenue qui comprend un conteneur où il y a un certain nombre de boulettes, le diamètre moyen d'une boulette ne dépassant pas 1 mm, chaque boulette comprenant un revêtement de quinidine sur une graine de nonpareil, les nonpareils ainsi enduits de quinidine étant enrobés d'un mélange de 2 1/3 à 9 parties en poids d'éthylcellulose pour 1 partie en poids d'hydroxypropylcellulose, la somme de ces boulettes dans un conteneur formant une seule dose de quinidine.

2. Forme de dosage de quinidine à libération entretenue de la revendication 1 où ladite quinidine est sous la forme de bisulfate de quinidine.

3. Forme de dosage de quinidine à libération entretenue de la revendication 1 où le poids de la quinidine est à peu près le double de celui du nonpareil.

4. Forme dosage de quinidine à libération entretenue de la revendication 1 où ledit conteneur est une capsule.

**Patentansprüche**

1. Dosierform zur Langzeitfreisetzung von Chinidin, umfassend einen Behälter, der eine große Anzahl Pellets enthält, wobei der durchschnittliche Pellet-Durchmesser nicht größer als 1 mm ist, und jedes Pellet einen Überzug aus Chinidin über Nonpareille-Kügelchen umfaßt, und die auf diese Weise Chinidin-beschichteten Kügelchen darauf aufgezogen werden mit einer Mischung aus 2 1/3 bis 9 Gewichtsteilen Ethylcellulose auf einen Gewichtsteil Hydroxypropylcellulose, wobei die Gesamtzahl an derartigen Pellets in einem Behälter eine einzelne Chinidin-Dosiereinheit bildet.

2. Dosierform zur Langzeitfreisetzung von Chinidin nach Anspruch 1, worin das Chinidin in Form von Chinidinbisulfat vorliegt.

3. Dosierform zur Langzeitfreisetzung von Chinidin nach Anspruch 1, worin das Gewicht des Chinidins ungefähr doppelt so groß ist wie das der Kügelchen.

4. Dosierform zur Langzeitfreisetzung von Chinidin nach Anspruch 1, worin der Behälter eine Kapsel ist.